# EUROPEAN PATENT APPLICATION

(11) **EP 1 700 589 A2**
(43) Date of publication of application: **13.09.2006**
(21) Application number: 06003329.7
(22) Date of filing: 18.02.2006
(51) Int. Cl.: A61F 13/535, A61F 13/15

(54) **Method of making absorbent core structures**

(30) Priority: 11.03.2005 US 906910
(71) Applicant: NORDSON CORPORATION, Westlake, Ohio 44145-1119 (US)
(72) Inventor: Bentley, Rachelle, Cumming Georgia 30040 (US); Crane, Patrick L., Dawsonville Georgia 30534 (US); Bernal, Stephen D., Cincinnati Ohio 45240 (US); Davis, James H., Amelia Ohio 45102 (US); Malakouti, Nezam, Loveland Ohio 45140 (US)
(74) Representative: Eisenführ, Speiser & Partner

(57) **Abstract**

A method of making an absorbent core structure includes meltspinning at least one layer of fibrous material. A first amount of superabsorbent material is deposited on the layer of fibrous material. A first portion of the layer of fibrous material is folded over the first amount of superabsorbent material, A second amount of superabsorbent material is deposited on the layer of fibrous material. A second portion of the layer of fibrous material is folded over the second amount of superabsorbent material. Additional embodiments involve rolling the fibrous material and/or densifying one of the layers relative to the other.

## Description

### FIELD OF THE INVENTION

The present invention relates to absorbent core structures for disposable absorbent articles. More specifically, the present invention relates to absorbent core structures constructed of fibrous materials.

### BACKGROUND OF THE INVENTION

Disposable absorbent articles having absorbent core structures are well known in the art. Furthermore, it is well known that such absorbent core structures have at least three functional regions, namely, an acquisition region, a distribution region, and a storage region. While such regions are known, the design of absorbent core structures having said regions is limited by current methods of manufacture and current material selections.

One such conventional absorbent core structure includes the use of cellulosic materials. While the use of cellulosic materials provide satisfactory acquisition and distribution, often cellulosic core structures suffer from having poor wet integrity (i.e., has poor structural integrity when wet). In an effort to improve the wet integrity of such cellulosic core structures, the incorporation of expensive binders is often used. Another known problem when using cellulosic materials is the presence of knots and fines which are unsatisfactorily shaped fibers that negatively impact the core properties (e.g., efficacy, cost).

Another such conventional absorbent core structure includes the use of synthetic meltblown fibers. While the use of synthetic meltblown fibers provides satisfactory wet integrity, the resulting core structure is often limited in design. For example, synthetic meltblown fibers are generally small in diameter (e.g., 2-9 microns); thus, the resulting core structure would generally have poor acquisition properties. Further, these smaller fibers tend to be weak thus not permitting the creation of post-hydrated void areas. Additionally, synthetic meltblown core structures often require the use of expensive binders.

It is also well known that conventional absorbent core structures for use in disposable absorbent articles may be made of discrete, multiple layers of materials. Further, it is well known that said layers may consist of different types of materials. For example, a conventional absorbent article may be made of: (a) a top layer which serves as an acquisition region for more immediate absorption of exudate from the wearer, (b) an intermediate layer which serves as a distribution region for the intended transportation of exudate within the absorbent core structure (e.g., move exudate longitudinally or laterally for greater utilization of diaper) and (c) a bottom layer which serves as a storage region for more long-term storage of exudate.

What is needed is an absorbent core structure made of fibrous material in which properties of the acquisition region, distribution region, and storage region can be easily varied in the vertical and/or horizontal direction.

### SUMMARY OF THE INVENTION

An absorbent core structure having at least one acquisition region, at least one distribution region, and at least one storage region. The acquisition region being constructed from a fibrous material. The acquisition region having a relatively low density from about 0.018 g/cc to about 0.20 g/cc. The at least one distribution region being constructed from the fibrous material. The distribution region being consolidated to have a relatively medium density from about 0.024 g/cc to about 0.45 g/cc. The distribution region being in fluid communication with said acquisition region. The storage region being constructed from the fibrous material. The storage region being consolidated to have a relatively high density from about 0.030 g/cc to about 0.50 g/cc. The storage region being in fluid communication with the distribution region. The fibrous material being folded to form said absorbent core structure. The fibrous material may be selected from the group consisting of polypropylene, polyethylene, polyester, polyvinyl alcohol, polyvinyl acetate, starch, cellulose acetate, polybutane, rayon, urethane, Kraton™, polylactic acid, cotton, Lyocell™, biogradeable polymers, any other material which is suitable for forming a fiber, and combinations thereof. The absorbent core structure may also include a superabsorbent material, such as a superabsorbent polymer (SAP) and/or other material with superabsorbent properties. The superabsorbent material may be deposited onto at least one of said storage regions.

An absorbent core structure having at least one acquisition region, at least one distribution region, and at least one storage region. The acquisition region being constructed from a fibrous material. The acquisition region having a relatively low density from about 0.018 g/cc to about 0.20 g/cc. The at least one distribution region being constructed from the fibrous material. The distribution region being consolidated to have a relatively medium density from about 0.024 g/cc to about 0.45 g/cc. The distribution region being in fluid communication with the acquisition region. The at least one storage region being constructed from the fibrous material. The storage region being consolidated to have a relatively high density from about 0.030 g/cc to about 0.50 g/cc. The storage region being in fluid communication with the distribution region. The fibrous material being rolled to form the absorbent core structure. The fibrous material may be selected from the group consisting of polypropylene, polyethylene, polyester, polyvinyl alcohol, polyvinyl acetate, starch, cellulose acetate, polybutane, rayon, urethane, Kraton™, polylactic acid, cotton, Lyocell™, biogradeable polymers, any other material which is suitable for forming a fiber, and combinations thereof. The absorbent core structure may also include a superabsorbent material, such as an SAP. The SAP may be deposited onto the at least one of the storage region.

An absorbent core structure having at least one acquisition region, at least one distribution region, and at least one storage region. The acquisition region being constructed from a first fibrous material. The first fibrous material having a relatively low density from about 0.018 g/cc to about 0.20 g/cc. The at least one distribution region being constructed from a second fibrous material. The distribution region being in fluid communication with the acquisition region. The second fibrous material having a relatively medium density from about 0.024 g/cc to about 0.45 g/cc. The at least one storage region being constructed from a third fibrous material. The storage region being in fluid communication with the distribution region. The third fibrous material having a relatively high density from about 0.030 g/cc to about 0.50 g/cc. The fibrous materials being layered to form the absorbent core structure. The fibrous materials may or mat not be constructed of substantially the same type of material. The fibrous materials may be selected from the group consisting of polypropylene, polyethylene, polyester, polyvinyl alcohol, polyvinyl acetate, starch, cellulose acetate, polybutane, rayon, urethane, Kraton™, polylactic acid, cotton, Lyocell™, biogradeable polymers, any other material which is suitable for forming a fiber, and combinations thereof. The absorbent core structure may also include a superabsorbent material, such as a SAP. The SAP may be deposited onto at least one of said storage region.

The invention further contemplates various methods of making an absorbent core structure, such as for use in a disposable hygienic product. In general, the methods involve the use of at least one layer of fibrous material and a superabsorbent material, such as those formed from various polymers and/or other materials. In the preferred manners of carrying out the methods of this invention, at least one layer of fibrous material is initially deposited on a moving collector, such as a conveying element formed from wire. The fibers are formed by a meltspinning process, such as a meltblowing and/or spunbonding process. In one illustrative method, a first amount of the superabsorbent material is deposited on the layer of fibrous material downstream from at least one meltspinning station. A first portion of the layer of fibrous material is folded over the superabsorbent material. A second amount of superabsorbent material is deposited on the layer of fibrous material, and a second portion of the layer of fibrous material is folded over the second amount of superabsorbent material.

The method can further comprise depositing the second amount of superabsorbent material on a side of the layer of fibrous material opposite to the first amount of superabsorbent material. It should also be understood that the various depositions of the superabsorbent material may be formulated of the same composition of superabsorbent material or different compositions of superabsorbent material depending on the needs of the application. The method may further involve densifying at least a portion of the layer of fibrous material. This can, for example, assist with fluid acquisition speed and containment of the superabsorbent material.

In another embodiment or aspect, the first and second amounts of superabsorbent material are deposited on a surface of the layer of fibrous material in spaced apart relation to define a space on the surface therebetween. In this aspect, the first portion of the layer of fibrous material may be folded over the space formed between the first and second amounts of superabsorbent material. The second portion of the layer of fibrous material may then be folded over the space and partially over the first portion to form a higher density region of the fibrous material in the space. The layer of fibrous material may be densified in a region generally defined by the surface having the first and second amounts of superabsorbent material deposited thereon.

In another embodiment of the disclosed methods, the superabsorbent material is deposited on the layer of fibrous material and the layer of fibrous material is rolled over the superabsorbent material to position the superabsorbent material between at least two portions of the layer of fibrous material. At least one of the portions of the layer of fibrous material may be densified. In a further aspect, the layer of fibrous material may be rolled over the superabsorbent material to form at least two layers of the superabsorbent material between at least three portions of the layer of fibrous material. The rolled layer of fibrous material may be reshaped into a form having a generally rectangular cross section, for example, which is more appropriate for the manufacture of products such as disposable hygienic articles.

In another embodiment of the inventive methods, at least first and second layers of the same or different fibrous materials are used and contain a superabsorbent material therebetween. More specifically, for example, one general method involves depositing a first amount of superabsorbent material on the first layer of fibrous material, placing the second layer of fibrous material over the first amount of superabsorbent material, and densifying at least a portion of one of the first and second layers relative to the other of the first and second layers. The method may further involve depositing a second amount of superabsorbent material on the second layer of fibrous material, and placing a third layer of fibrous material over the second amount of superabsorbent material. As another aspect, the method can further comprise densifying at least two of the first, second and third layers of fibrous material relative to each other and relative to the remaining layer of fibrous material. As mentioned, the different layers may be formed from the same type of fiber, or the fibers in one or more layers may have different properties than the fibers of one or more of the remaining layers.

Various additional features, advantages and objectives of the invention will become more readily apparent to those of ordinary skill in the art upon review of the following detailed description of the preferred embodiments taken in conjunction with the accompanying drawings.

### BRIEF DESCRIPTION OF THE DRAWINGS

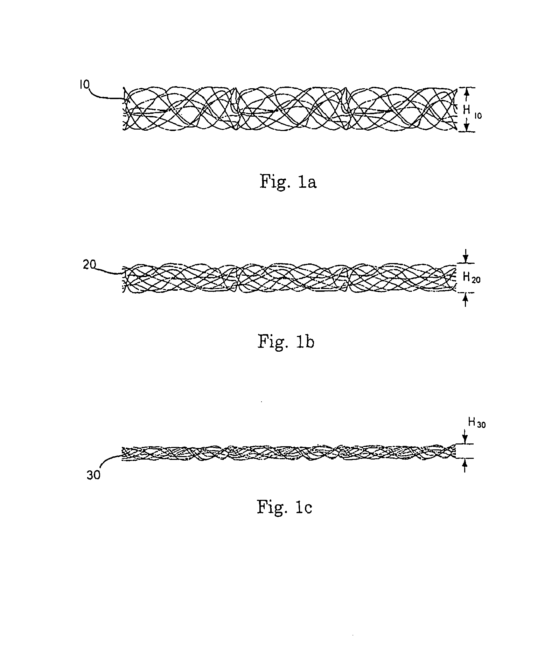

FIG. 1a depicts a first layer of fibrous material having a relatively low density and a relatively high caliper;

FIG. 1 b depicts a second layer of fibrous material having a relatively medium density and a relatively medium caliper;

FIG. 1c depicts a third layer of fibrous material having a relatively high density and a relatively low caliper;

FIG. 2a shows an exemplary first step of an exemplary manufacturing process to make a first exemplary embodiment of the present invention;

FIG. 2b shows first layer of fibrous material being folded around SAP;

FIG. 2c shows another deposition of SAP being placed upon the folded portion of first layer fibrous material;

FIG. 2d-1 shows an exemplary resulting product wherein first layer fibrous material is folded over the second deposition of SAP and the density of said layer remains substantially the same;

FIG. 2d-2 shows another exemplary resulting product wherein the first layer of fibrous material is densified such that said layer now has a density which is relatively high;

FIG. 2d-3 shows yet another exemplary resulting product wherein the first layer of fibrous material is densified along its lower portion such that a bottom layer has a density which is relatively high while the middle layer and top layer remain relatively lofted;

FIG. 2d-4 shows yet another exemplary resulting product wherein the first layer of fibrous material is densified in a gradient pattern such that the bottom layer has a relatively high density, the middle layer has a relatively medium density and the top layer has a relatively low density;

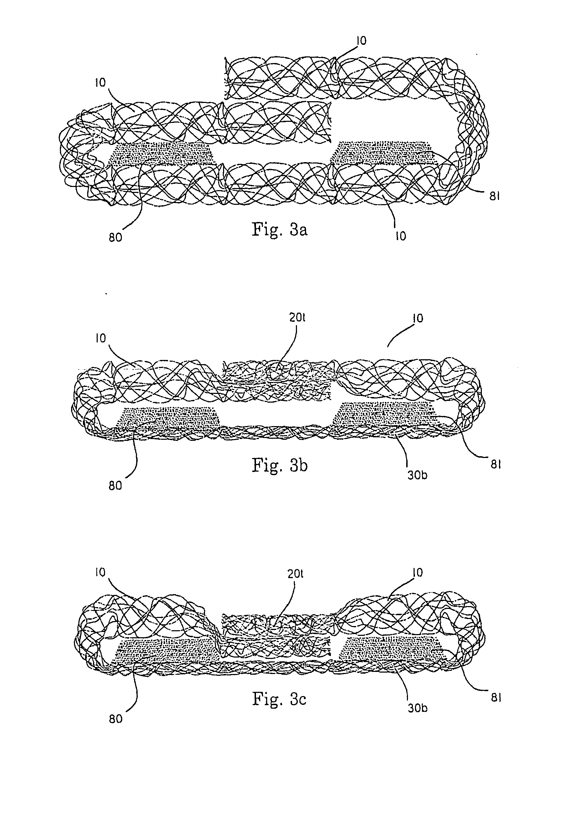

FIG. 3a shows an exemplary embodiment of the present invention wherein a first layer of fibrous material is folded in an overlapping fashion around two spaced apart depositions of SAP;

FIG. 3b shows the product of FIG. 3a undergoing densification such that the bottom layer now has a relatively high density and the central portion of the top layer now has a medium density;

FIG. 3c shows a product of FIG. 3b undergoing even further densification;

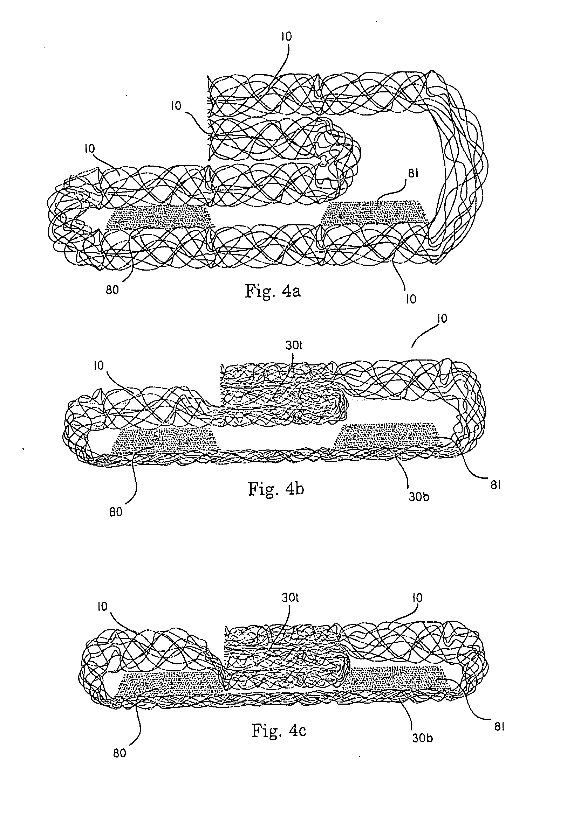

FIG. 4a shows an exemplary embodiment of the present invention wherein a first layer of fibrous material is folded in a butt-joint fashion around spaced apart depositions of SAP;

FIG. 4b shows the product of FIG. 4a undergoing densification such that the bottom layer now has a relatively high density and the central portion of the top layer now has a relatively higher density;

FIG. 4c shows the product of FIG. 4b undergoing even further densification such that the central portion of the top layer now fills the void between the spaced apart depositions of SAP;

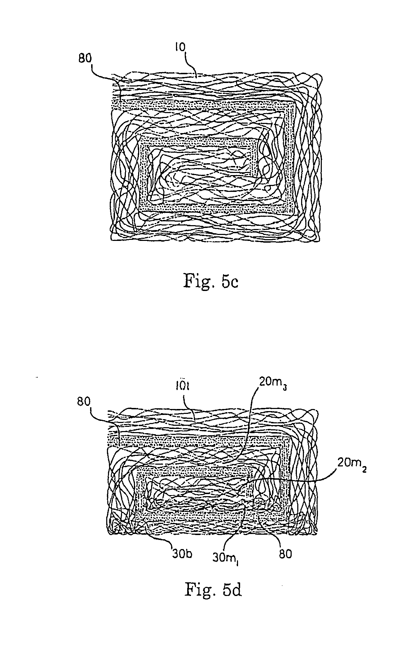

FIG. 5a shows an exemplary process wherein SAP particles are deposited from an SAP applicator onto a layer of fibrous material;

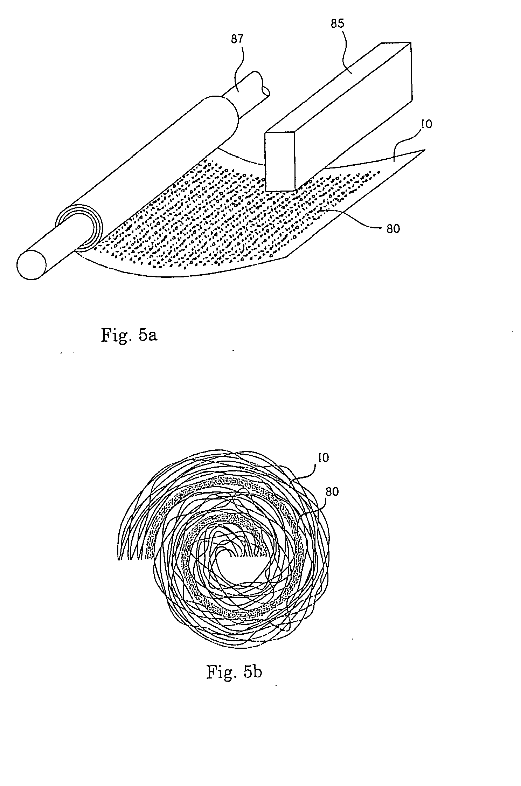

FIG. 5b depicts the rolled combination of fibrous material and SAP being removed from mandrel;

FIG. 5c shows the rolled combination from FIG. 5b being reshaped;

FIG. 5d shows the substantially rectangular combination fibrous material and SAP undergoing a gradient of densification;

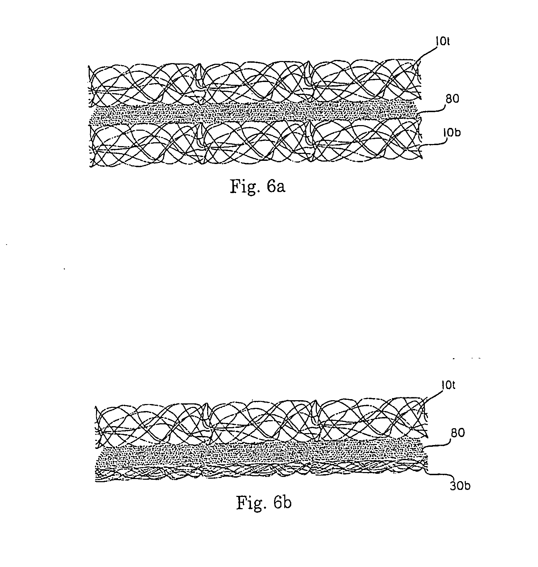

FIG. 6a shows a bottom layer of fibrous material and a top layer of fibrous material, both having a relatively low density;

FIG. 6b shows the product of FIG. 6a undergoing densification such that the bottom layer now has a relatively high density, while the top layer still has a relatively low density;

**FIG.** 7a shows a bottom layer of fibrous material, a middle layer of fibrous material and a top layer of fibrous material, each having a relatively low density.

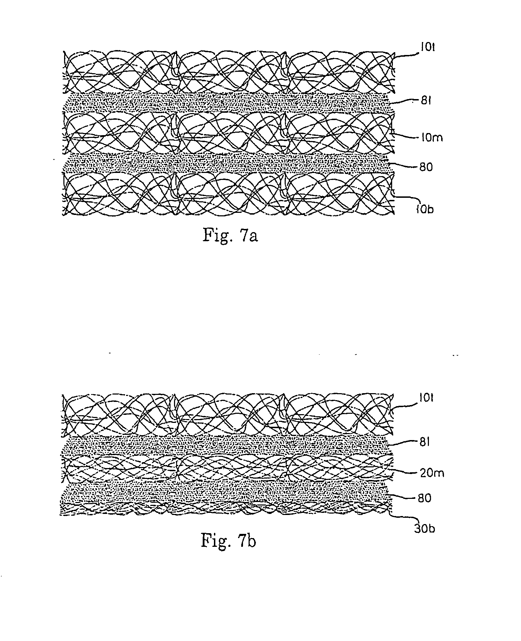

FIG. 7b shows the product of FIG. 7a undergoing a gradient of densification;

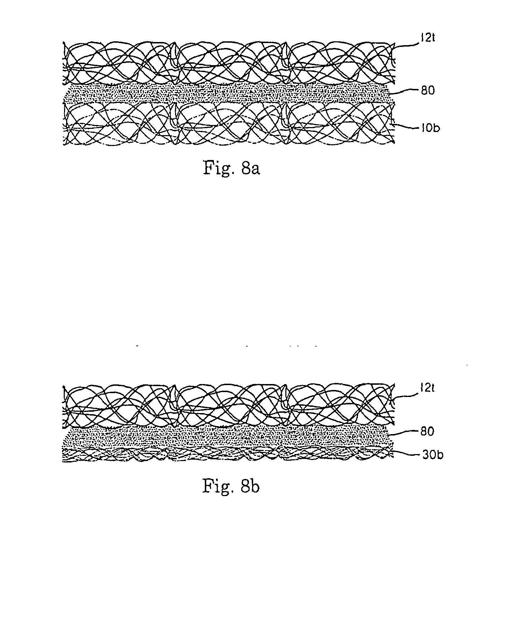

FIG. 8a shows a bottom layer of a first fibrous material and a top layer of a second fibrous material, wherein said layers have different properties;

FIG. 8b shows the product of FIG. 8a undergoing densification;

FIG. 9a shows a bottom layer of a first fibrous material, a second layer of a second fibrous material and a top layer of said first fibrous material, each having a relatively low density;

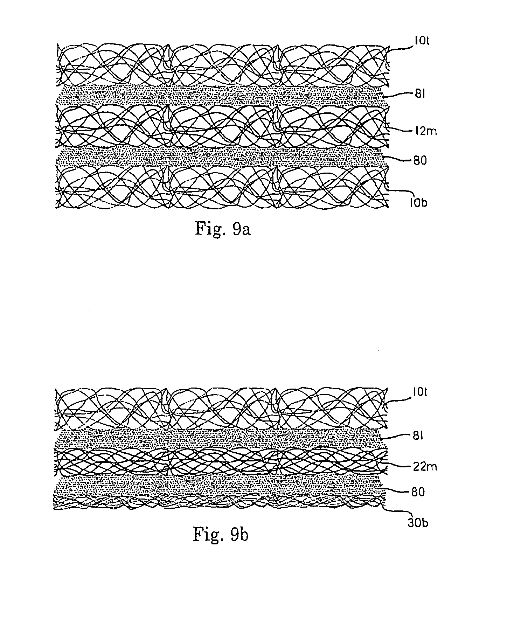

FIG. 9b shows the product of FIG. 9a undergoing densification;

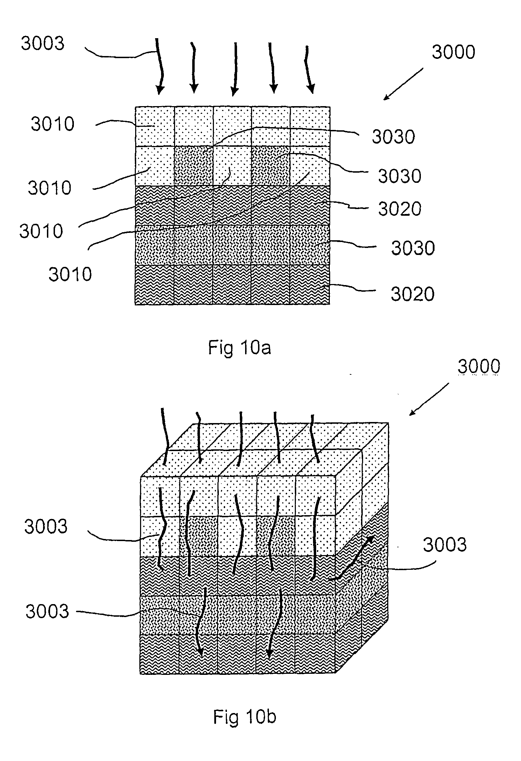

FIG. 10a shows a two-dimensional schematic view of an absorbent core having acquisition regions, distribution regions and storage regions being selectively placed throughout the core design;

FIG. 10b shows a three-dimensional schematic of FIG. 10a with fluid moving therein;

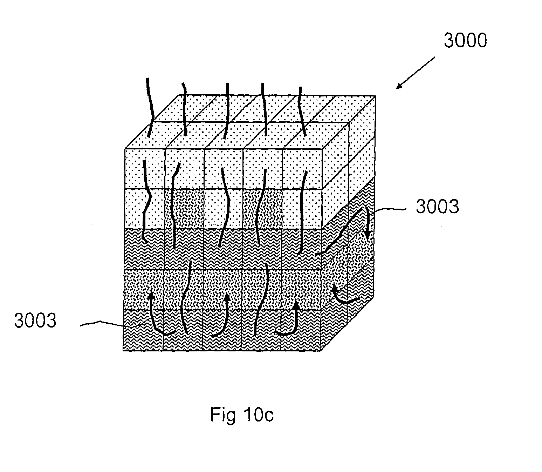

FIG. 10c shows a three-dimensional schematic of FIG. 10b with fluid moving further therein; and

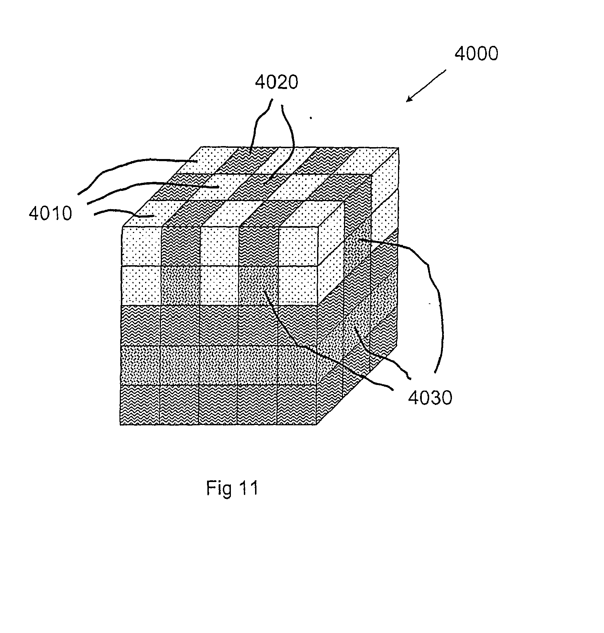

FIG. 11 shows a three-dimensional schematic view of another absorbent core having acquisition regions, distribution regions and storage regions vary in their three-dimensional placement.

### DETAILED DESCRIPTION OF THE EMBODIMENTS

Various definitions of terms used herein are provided as follows:

The term "absorbent article" herein refers to devices which absorb and contain body exudates and, more specifically, refers to devices which are placed against or in proximity to the body of the wearer to absorb and contain the various exudates discharged from the body, such as: incontinence briefs, incontinence undergarments, absorbent inserts, diaper holders and liners, feminine hygiene garments and the like. The absorbent article may have an absorbent core having a garment surface and a body surface; a liquid permeable topsheet positioned adjacent the body surface of the absorbent core; and a liquid impermeable backsheet positioned adjacent the garment surface of the absorbent core.

The term "disposable" is used herein to describe absorbent articles which generally are not intended to be laundered or otherwise restored or reused as absorbent articles (i.e., they are intended to be discarded after a single use and, preferably, to be recycled, composted or otherwise discarded in an environmentally compatible manner).

The term "diaper" herein refers to an absorbent article generally worn by infants and incontinent persons about the lower torso.

The term "pant", as used herein, refers to disposable garments having a waist opening and leg openings designed for infant or adult wearers.
A pant may be placed in position on the wearer by inserting the wearer's legs into the leg openings and sliding the pant into position about the wearer's lower torso. A pant may be preformed by any suitable technique including, but not limited to, joining together portions of the article using refastenable and/or non-refastenable bonds (e.g., seam, weld, adhesive, cohesive bond, fastener, etc.). A pant may be preformed anywhere along the circumference of the article (e.g., side fastened, front waist fastened). While the term "pant" is used herein, pants are also commonly referred to as "closed diapers", "prefastened diapers", "pull-on diapers", "training pants" and "diaper-pants". Suitable pants are disclosed in U.S. Patent No. 5,246,433, issued to Hasse, et al. on September 21, 1993; U.S. Patent No. 5,569,234, issued to Buell et al. on October 29, 1996; U.S. Patent No. 6,120,487, issued to Ashton on September 19, 2000; U.S. Patent No. 6,120,489, issued to Johnson et al. on September 19, 2000; U.S. Patent No. 4,940,464, issued to Van Gompel et al. on July 10, 1990; U.S. Patent No. 5,092,861, issued to Nomura et al. on March 3, 1992; U.S. Patent Application Serial No. 10/171,249, entitled "Highly Flexible And Low Deformation Fastening Device", filed on June 13, 2002; U.S. Patent No. 5,897,545, issued to Kline et al. on April 27, 1999; U.S. Patent No. 5,957,908, issued to Kline et al on September 28, 1999.

The term "machine direction (MD)" or "longitudinal" herein refers to a direction running parallel to the maximum linear dimension of the article and/or fastening material and includes directions within ±45° of the longitudinal direction.

The term "cross direction (CD)", "lateral" or "transverse" herein refers to a direction which is orthogonal to the longitudinal direction.

The term "joined" encompasses configurations whereby an element is directly secured to another element by affixing the element directly to the other element, and configurations whereby an element is indirectly secured to another element by affixing the element to intermediate member(s) which in turn are affixed to the other element.

As used herein the term "spunbond fibers" refers to small diameter fibers of substantially molecularly oriented polymeric material. Spunbond fibers are generally formed by extruding molten thermoplastic material as filaments from a plurality of fine, usually circular capillaries of a spinneret with the diameter of the extruded filaments then being rapidly reduced by an attenuation process. Spunbond fibers are generally not tacky when they are deposited onto a collecting surface and are generally continuous.

As used herein the term "spunbond material" refers to material made from spunbond fibers.

As used herein the term "meltblown fibers" means fibers of polymeric material which are generally formed by extruding a molten thermoplastic material through a plurality of fine, usually circular, die capillaries as molten threads or filaments into converging high velocity, usually hot, gas (e.g. air) streams which attenuate the filaments of molten thermoplastic material to reduce their diameter. Thereafter, the meltblown fibers can be carried by the high velocity gas stream and are deposited on a collecting surface to form a web of randomly dispersed meltblown fibers. Meltblown fibers may be continuous or discontinuous, are generally smaller than 10 microns in average diameter, and are generally tacky when deposited onto a collecting surface.

As used herein the term "polymer" generally includes but is not limited to, homopolymers, copolymers, such as for example, block, graft, random and alternating copolymers, terpolymers, etc. and blends and modifications thereof. Furthermore, unless otherwise specifically limited, the term "polymer" includes all possible spatial configurations of the molecule. These configurations include, but are not limited to isotactic, syndiotactic and random symmetries.

As used herein, "ultrasonic bonding" means a process performed, for example, by passing the fabric between a sonic horn and anvil roll.

As used herein the term "acquisition layer" or "acquisition region" means a fibrous material having a relatively low density from about 0.018 g/cc to about 0.20 g/cc and a relatively high caliper from about 0.41 mm to about 5.23 mm.

As used herein the term "distribution layer" or "distribution region" means a fibrous material having a relatively medium density from about 0.024 g/cc to about 0.45 g/cc and a relatively medium caliper from about 0.39 mm to about 4.54 mm.

As used herein the terms "storage layer" or "storage region" mean any region that contains superabsorbent polymer. Further, the terms mean a fibrous material having a relatively high density from about 0.030 g/cc to about 0.50 g/cc and a relatively low caliper 0.15 mm to about 3.96 mm.

As used herein the term "small diameter" describes any fiber with a diameter of less than or equal to 10 microns.

As used herein the term "large diameter" describes any fiber with a diameter of greater than 10 microns.

As used herein the term "superabsorbent" refers to a material that can absorb at least about 10 times its weight in fluid.

FIG. 1 a depicts a first layer of fibrous material 10 having a relatively low density from about 0.018 g/cc to about 0.20 g/cc) and a relatively high caliper from about 0.41 mm to about 5.23 mm (shown as H₁₀). The first layer fibrous material 10 having particular usefulness as an acquisition layer. FIG. 1 b depicts a second layer of fibrous material 20 having a relatively medium density from about 0.024 g/cc to about 0.45 g/cc and a relatively medium caliper from about 0.39 mm to about 4.54 mm (shown as H₂₀). The second layer of fibrous material 20 having particular usefulness as a distribution layer. FIG. 1c depicts a third layer of fibrous material 30 having a relatively high density from about 0.030 g/cc to about 0.50 g/cc and a relatively low caliper 0.15 mm to about 3.96 mm (shown as H₃₀).

Fibrous material 10 of each type of layer generally has a basis weight from about 5 gsm to about 1000 gsm. The fibers of fibrous material 10 may be made of a variety of suitable materials including, but not limited to, polypropylene, polyethylene, polyester, polyvinyl alcohol, polyvinyl acetate, starch, cellulose acetate, polybutane, rayon, urethane, Kraton™, polylactic acid, cotton, Lyocell™, biogradeable polymers, any other material which is suitable for forming a fiber, and combinations thereof. The fibrous fibers of the present invention may have a diameter from about 10 micron to about 600 microns, unlike conventional meltblown fibers which typically have a diameter from about 2 to about 9 microns. Having such a larger diameter allows for the creation of high density fibrous materials which provide the necessary void space for acquisition layers. Being able to modify the density is also necessary in order to provide distribution and storage areas. Such modification techniques include, but are not limited to, consolidation (e.g., nip rolls, vacuum while attenuating fibers in a manufacturing beam, etc.), calendering (e.g., nip rolls with heat), ultrasonic and through air bonding (as exampled in U.S. Patent No. 4,011,124).

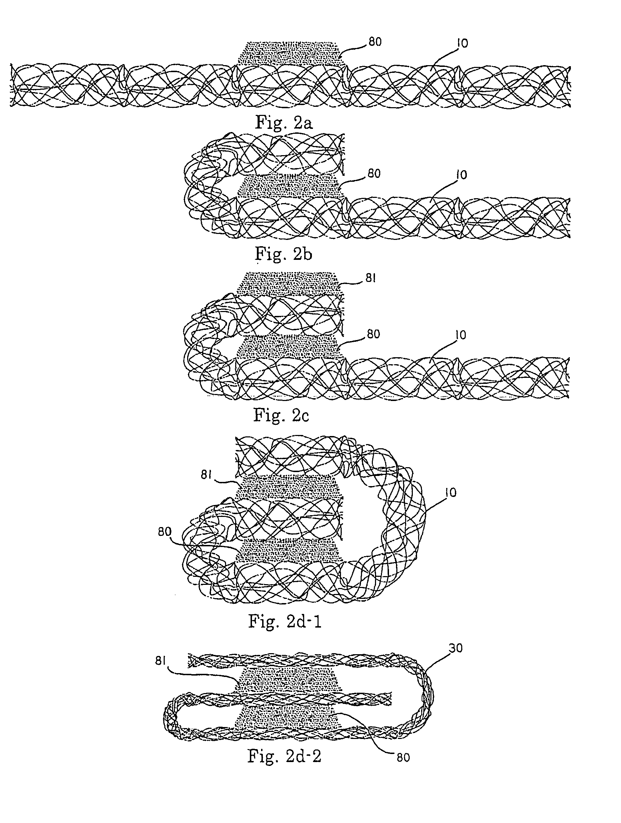

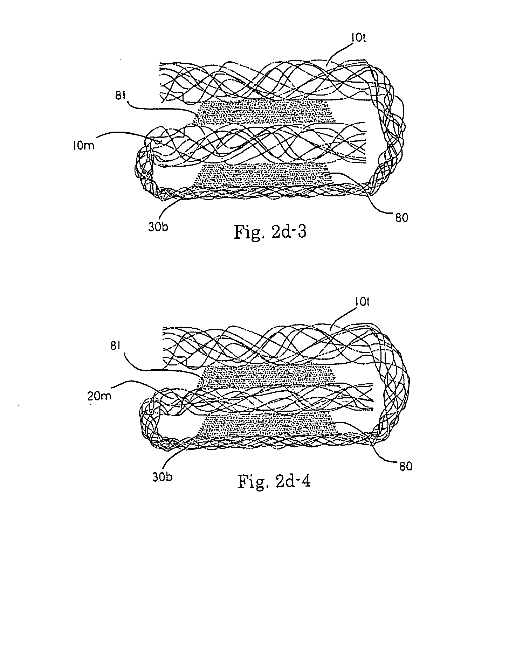

FIG. 2a shows an exemplary first step of an exemplary manufacturing process to make a first exemplary embodiment of the present invention. More specifically, a first layer of fibrous material 10 is first laid down. An exemplary width of fibrous material 10 may be about 300 mm. Next, a super absorbent polymer 80 (hereinafter "SAP") is placed/deposited upon the fibrous material 10. The deposition may be accomplished by any suitable technique including, but not limited to, conventional SAP metering systems. An exemplary SAP deposition amount may range from about 10 gsm to about 1000 gsm, preferably about 50 gsm to about 800 gsm. The exemplary deposition amount may correspond to a height from about 0.001 mm to about 3 mm. FIG. 2b shows first layer of fibrous material 10 being folded around the SAP 80. The folding may be accomplished by any suitable technique including, but not limited to, guiding surfaces (e.g., folding boards, belts, rollers, plates, idlers, etc.), drawing (e.g., applying tension via control points, etc.), pneumatics (e.g., vacuum, blown air, etc.) and electrostatic. It may be desirable to use adhesive for subsequent folding. FIG. 2c shows another deposition of SAP 81 being placed upon the folded portion of first layer fibrous material 10. FIG. 2d-1 shows an exemplary resulting product wherein first layer fibrous material 10 is folded over the second deposition of SAP 81 and the density of the layer remains substantially the same. Alternatively, FIG. 2d-2 shows another exemplary resulting product wherein the first layer of fibrous material 10 is densified such that the layer now has a density which is relatively high. Alternatively, FIG. 2d-3 shows yet another exemplary resulting product wherein the first layer of fibrous material 10 is densified along its lower portion such that a bottom layer 30b has a density which is relatively high while the middle layer 10m and top layer 10t remain relatively lofted. Alternatively, FIG. 2d-4 shows yet another exemplary resulting product wherein the first layer of fibrous material 10 is densified in a gradient pattern such that the bottom layer 30b has a relatively high density, the middle layer 20m has a relatively medium density and the top layer 10t has a relatively low density.

FIG. 3a shows an exemplary embodiment of the present invention wherein a first layer of fibrous material 10 is folded in an overlapping fashion around two spaced apart depositions of SAP 80, 81. FIG. 3b shows the product of FIG. 3a undergoing densification such that the bottom layer 30b now has a relatively high density and the central portion of the top layer 20t now has a medium density. Providing a bottom layer 30b with a high density helps to resist SAP from falling out below and also provides a lower distribution/storage region. An underneath distribution layer helps to distribute urine both laterally and/or longitudinally within the absorbent core so as to improve overall utilization of the SAP throughout the core and to improve acquisition performance of subsequent urine insults. FIG. 3c shows the product of FIG. 3b undergoing even further densification such that the central portion of the top layer 20t now fills the void between the spaced apart depositions of SAP 80, 81.

FIG. 4a shows an exemplary embodiment of the present invention wherein a first layer of fibrous material 10 is folded in a butt-joint fashion around spaced apart depositions of SAP 80, 81. FIG. 4b shows the product of FIG. 4a undergoing densification such that the bottom layer 30b now has a relatively high density and the central portion of the top layer 30t now has a relatively higher density. Providing such a central portion of the top layer 30t having a higher density helps to further distribute urine, particularly in the longitudinal direction so as to improve overall utilization of the SAP throughout the core and to improve acquisition performance of subsequent urine insults. FIG. 4c shows the product of FIG. 4b undergoing even further densification such that the central portion of the top layer 30t now fills the void between the spaced apart depositions of SAP 80, 81.

FIG. 5a shows an exemplary process wherein SAP particles 80 are deposited from an SAP applicator 85 onto a layer of fibrous material 10. Next, the combination of fibrous material 10 and SAP 80 are rolled about a mandrel 87 or any other like apparatus. FIG. 5b depicts the rolled combination of fibrous material 10 and SAP 80 being removed from mandrel 87. FIG. 5c shows the rolled combination from FIG. 5b being reshaped, for example, in a substantially rectangular geometry. FIG. 5d shows the substantially rectangular combination fibrous material 10 and SAP 80 undergoing a gradient of densification such that the bottom layer 30b now having a relatively high density, a first middle layer 30m₁ now having a relatively higher density, a second middle layer 20m₂ now having a relatively medium density, a third middle layer 20m₃ now having a relatively medium density and a top layer 10t still having a relatively low density. This particular embodiment provides the unique benefit of two or more acquisition and/or distribution layers and two or more storage areas. Such a unique design is particularly useful to protect against subsequent urine insults.

FIG. 6a shows a bottom layer of fibrous material 10b and a top layer of fibrous material 10t, both having a relatively low density. Additionally, a layer of SAP 80 is deposited between the layers. FIG. 6b shows the product of FIG. 6a undergoing densification such that the bottom layer 30b now has a relatively high density, while the top layer 10t still has a relatively low density. The multiple layers of this particular embodiment may be formed by different manufacturing beams. One skilled in the art may recognize that particular care (e.g., slower manufacturing rates) may be needed when laying additional fiber layers on top of SAP (e.g., displacement air may move SAP about).

FIG. 7a shows a bottom layer of fibrous material 10b, a middle layer of fibrous material 10m and a top layer of fibrous material 10t, each having a relatively low density. Additionally, a first layer of SAP 80 is deposited between the bottom and middle layers. A second layer of SAP 81 is deposited between the middle and top layers. FIG. 7b shows the product of FIG. 7a undergoing a gradient of densification such that the bottom layer 30b now has a relatively high density, the middle layer 20m now having a relatively medium density and the top layer 10t still having a relatively low density. The multiple layers of this particular embodiment may be formed by different manufacturing beams. One skilled in the art may recognize that particular care (e.g., slower manufacturing rates) may be needed when laying additional fiber layers on top of SAP (e.g., displacement air may move SAP about).

FIG. 8a shows a bottom layer of a first fibrous material 10b and a top layer of a second fibrous material 12t, wherein the layers have different properties (e.g., material types, fiber diameters, fiber shapes, melt points, etc.). For example, first fibrous material 10b may be made of polypropylene which is inexpensive, easy to modify hydrophilicity and easy to process (e.g., manufacturing beams may sit idle for many hours) and second fibrous material 12t may be made of polyester which is less susceptible to stress relaxation which is helpful in resisting packaging compression. In another example, second fibrous material 12t may be made of a fiber having a relatively larger diameter in order to create void spaces and to decrease surface area (urine would be less likely to distribute and more likely to remain in place); whereas, first fibrous material 10b may be made of a fiber having a relatively smaller diameter to improve distribution and/or storage. In yet another example, first fibrous material 10b may be made of a fiber having a non-circular cross-section (e.g., pentalobal, trilobal, 4dg from Eastman, etc.) in order to increase surface area for improved acquisition and distribution; whereas, second fibrous material 12t may be made of a fiber having a substantially circular cross-section in order to decrease surface area. In yet another example, bicomponent materials may be used, particularly with the use of through air bonding, in order to improve overall strength. Additionally, a layer of SAP 80 is deposited between the layers. FIG. 8b shows the product of FIG. 8a undergoing densification such that the bottom layer 30b now has a relatively high density and the top layer 12t still having a relatively low density. The multiple layers of this particular embodiment may be formed by different manufacturing beams. One skilled in the art may recognize that particular care (e.g., slower manufacturing rates) may be needed when laying additional fiber layers on top of SAP (e.g., displacement air may move SAP about).

FIG. 9a shows a bottom layer of a first fibrous material 10b, a second layer of a second fibrous material 12m and a top layer of the first fibrous material 10t, each having a relatively low density. Further, the first and second material may have different properties (e.g., different materials, different diameters, different melt points, etc.). Additionally, a first layer of SAP 80 is deposited between the bottom and middle layers. A second layer of SAP 81 is deposited between the middle and top layers. FIG. 9b shows the product of FIG. 9a undergoing densification such that the bottom layer 30b now has a relatively high density, the middle layer 22m now has a relatively medium density and the top layer 12t still having a relatively low density. The multiple layers of this particular embodiment may be formed by different manufacturing beams. One skilled in the art may recognize that particular care (e.g., slower manufacturing rates) may be needed when laying additional fiber layers on top of SAP (e.g., displacement air may move SAP about).

Referring now to FIG. 10a, a two-dimensional schematic is shown to depict one of the benefits of the present invention. More specifically, the novel aspects of the present invention provide for the creation of novel core structure designs. For instance, FIG. 10a shows a two-dimensional schematic view of an absorbent core 3000 having acquisition regions 3010, distribution regions 3020 and storage regions 3030 being selectively placed throughout the core design. Such a designs provides for novel fluid management.

It is well known that conventional absorbent core structures for use in disposable absorbent articles may be made of multiple layers of materials. Further, it is well known that the layers may consist of different types of materials. For example, a conventional absorbent article may be made of: (a) a top layer which serves as an acquisition region for more immediate absorption of exudate from the wearer, (b) an intermediate layer which serves as a storage region for more long-term storage of exudate and (c) a bottom layer which serves as a distribution region for the intended transportation of exudate within the absorbent core structure (e.g., move exudate longitudinally or laterally for greater utilization of diaper). Not only does the present invention provide interlayer fluid communication, but it provides three-dimensional fluid management as depicted in the series of FIGS. 10a-10c, wherein the fluid 3003 is moved in accordance with the core design principles disclosed herein. Lastly, the core structure may be designed to have its regions (i.e., acquisition regions 4010, distribution regions 4020 and storage regions 4030) vary in their three-dimensional placement as depicted by absorbent core 4000 in FIG. 11.

All documents cited in the Detailed Description of the Invention are, in relevant part, incorporated herein by reference; the citation of any document is not to be construed as an admission that it is prior art with respect to the present invention.

While particular embodiments of the present invention have been illustrated and described, it would be obvious to those skilled in the art that various other changes and modifications can be made without departing from the spirit and scope of the invention. It is therefore intended to cover in the appended claims all such changes and modifications that are within the scope of this invention.

For example, one skilled in the art would appreciate varying degrees of consolidation.

## Claims

1. A method of making an absorbent core structure, comprising:
meltspinning at least one layer of fibrous material,
depositing a first amount of superabsorbent material on the layer of fibrous material,
folding a first portion of the layer of fibrous material over the first amount of superabsorbent material,
depositing a second amount of superabsorbent material on the layer of fibrous material, and
folding a second portion of the layer of fibrous material over the second amount of superabsorbent material.

2. The method of claim 1, wherein depositing the second amount of superabsorbent material further comprises:
depositing the second amount of superabsorbent material on a side of the layer of fibrous material opposite to the first amount of superabsorbent material.

3. The method of claim 1, further comprising:
densifying at least a portion of the layer of fibrous material.

4. The method of claim 1, further comprising:
depositing the first and second amounts of superabsorbent material on a surface of the layer of fibrous material in spaced apart relation to define a space on the surface therebetween.

5. The method of claim 4, further comprising:
folding the first portion of the layer of fibrous material over the space, and
folding the second portion of the layer of fibrous material over the space and partially over the first portion to form a higher density region of the fibrous material in the space between the first and second amounts of superabsorbent material.

6. The method of claim 5, further comprising:
densifying the layer of fibrous material in a region generally defined by the surface having the first and second amounts of superabsorbent material deposited thereon.

7. A method of making an absorbent core structure, comprising:
meltspinning at least one layer of fibrous material,
depositing a superabsorbent material on the layer of fibrous material, and
rolling the layer of fibrous material over the superabsorbent material to position the superabsorbent material between at least two portions of the layer of fibrous material.

8. The method of claim 7, further comprising:
densifying at least one of the portions of the layer of fibrous material.

9. The method of claim 7, further comprising;
rolling the layer of fibrous material over the superabsorbent material to form at least two layers of the superabsorbent material between at least three portions of the layer of fibrous material.

10. The method of claim 7, further comprising:
reshaping the rolled layer of fibrous material into a form having a generally rectangular cross section.

11. A method of making an absorbent core structure from first and second layers of fibrous material and a superabsorbent material, comprising:
meltspinning first and second layers of fibrous material,
depositing a first amount of superabsorbent material on the first layer of fibrous material,
placing the second layer of fibrous material over the first amount of superabsorbent material, and
densifying at least a portion of one of the first and second layers relative to the other of the first and second layers.

12. The method of claim 11, further comprising:
depositing a second amount of superabsorbent material on the second layer of fibrous material, and
placing a third layer of fibrous material over the second amount of superabsorbent material.

13. The method of claim 12, further comprising:
densifying at least two of the first, second and third layers of fibrous material relative to each other and relative to the remaining layer of fibrous material.

14. The method of claim 11, wherein the first and second layers of fibrous material are respectively formed from fibers having different properties.
